# EUROPÄISCHE PATENTSCHRIFT

(11) **EP 3 439 627 B1**
(45) Veröffentlichungstag und Bekanntmachung des Hinweises auf die Patenterteilung: **18.10.2023**
(21) Anmeldenummer: 17716523.0
(22) Anmeldetag: 07.04.2017
(51) Int. Cl.: A61K 8/81, A61P 1/02, A61Q 11/02, A61Q 11/00, A61K 31/785, A61K 9/00, A61K 8/9789, A61P 15/02, A61P 29/00, A61K 9/06, A61Q 5/02, A61Q 17/00, A61Q 19/00, A61Q 19/08, A61Q 19/10

(54) **MITTEL ZUR VERWENDUNG BEI ENTZÜNDLICHEN ZUSTÄNDEN DER SCHLEIMHÄUTE**
COMPOSITION FOR USE FOR INFLAMMATORY CONDITIONS OF THE MUCOSA
COMPOSITION POUR UTILISATION CONTRE DES INFLAMMATIONS DES MUQUEUSES

(30) Priorität: 08.04.2016 DE 102016205950
(43) Veröffentlichungstag der Anmeldung: 13.02.2019
(73) Patentinhaber: Vermögensverwaltung D. Th. Seidel GmbH, 69198 Schriesheim (DE)
(72) Erfinder: SEIDEL, Dietrich, 82340 Feldafing (DE)
(74) Vertreter: Weickmann & Weickmann PartmbB
(86) Internationale Anmeldenummer: PCT/EP2017/058429
(87) Internationale Veröffentlichungsnummer: WO 2017/174796

(56) Entgegenhaltungen:
- EP-A2- 1 053 747
- WO-A1-2012/123363
- DE-A1- 2 920 071
- DE-A1- 3 930 206
- DE-T2- 69 011 353
- DE-U1-202014 009 184
- GB-A- 2 021 949
- SU-A1- 825 074
- STROEHLEIN J R: "Treatment of Clostridium difficile infection", CURRENT TREATMENT OPTIONS IN GASTROENTEROLOGY, Bd. 7, Nr. 3, 1. Juni 2004 (2004-06-01), Seiten 235-239, XP009194815,
- KELLY RANKIN ET AL: "Treatment of Cyanobacterial (Microcystin) Toxicosis Using Oral Cholestyramine: Case Report of a Dog from Montana", TOXINS, Bd. 5, Nr. 6, 29. Mai 2013 (2013-05-29), Seiten 1051-1063, XP055386310, CH ISSN: 2072-6651, DOI: 10.3390/toxins5061051
- KERKADI ABDELHAMID ET AL: "Dietary cholestyramine reduces ochratoxin A-induced nephrotoxicity in the rat by decreasing plasma levels and enhancing fecal excretion of the toxin", JOURNAL OF TOXICOLOGY AND ENVIRONMENTAL HEA, TAYLOR 6 FRANCIS, US, Bd. 53, Nr. 3, 6. Februar 1998 (1998-02-06), Seiten 231-250, XP009125648, ISSN: 0098-4108, DOI: 10.1080/009841098159367
- TAYLOR ET AL: "Retention of oral microorganisms on cobalt-chromium alloy and dental acrylic resin with different surface finishes", JOURNAL OF PROSTHETIC DENTIS, ELSEVIER, AMSTERDAM, NL, Bd. 80, Nr. 5, 1. November 1998 (1998-11-01), Seiten 592-597, XP005693490, ISSN: 0022-3913, DOI: 10.1016/S0022-3913(98)70037-X

## Beschreibung

Die vorliegende Erfindung betrifft eine Zusammensetzung zur Verwendung bei der Vorbeugung oder/und Behandlung von entzündlichen Zuständen oder/und Erkrankungen von Schleimhäuten, insbesondere im Bereich der Mundhöhle. Der Gegenstand der vorliegenden Erfindung ist in den Ansprüchen definiert. Im Folgenden beschriebene Behandlungsmethoden beziehen sich auf Verbindungen / Zusammensetzungen der vorliegenden Erfindung zur Verwendung in einem Verfahren zur therapeutischen Behandlung eines menschlichen (tierischen) Körpers.

Die Mundhöhle stellt den Beginn des Verdauungstrakts dar. Sie ist mit einer Schleimhaut, der sogenannten Mundschleimhaut, ausgekleidet und wird von einer Vielzahl von Mikroorganismen besiedelt, welchen sich dort günstige Lebensbedingungen bieten. Zum einen ist die Mundhöhle ein Raum mit konstanter und relativ hoher Temperatur, der sich auch durch eine hohe Feuchtigkeit auszeichnet. Zum anderen erfolgt die Nahrungsaufnahme über die Mundhöhle, wodurch in diesem Raum auch ein kontinuierlicher Nährstoffeintrag gegeben ist. Bedingt durch das Vorhandensein von schwer zugänglichen Engstellen, z.B. zwischen den Zähnen, kann es zu einer Ansammlung von Speiseresten kommen, welche einen günstigen Nährboden für mikrobielles Wachstum darstellen. Die Mundhöhle bietet somit geeignete Bedingungen für die Vermehrung von Mikroorganismen.

Bei einem gesunden Menschen steht die Mikroflora der Mundhöhle in einem natürlichen Gleichgewicht. So kommt es im Normalfall weder zu einer übermäßigen Vermehrung bestimmter Spezies von Mikroorganismen, noch zu einer Besiedelung durch pathogene Mikroorganismen von außen. In Fällen, in denen die Mundschleimhaut ihre natürliche Barrierefunktion nicht mehr erfüllen kann, z.B. infolge von Verletzungen oder als Folge von Entzündungen der Mundschleimhaut, können Mikroorganismen mit tiefer liegendem Gewebe in Kontakt kommen und eine zunächst meist lokale Infektion verursachen. Infolgedessen werden Abwehrmechanismen des Organismus in Gang gesetzt oder verstärkt, und es kommt zu einer zunächst meist lokalen Entzündungsreaktion. Diese kann sich auf angrenzendes Gewebe erstrecken und auch zu chronischen Entzündungszuständen führen. In ungünstigen Fällen kann sich eine solche lokale Infektion sogar systemisch ausbreiten. Bekannt ist auch ein Zusammenhang zwischen Infektionen der Mundhöhle mit gleichzeitiger Entwicklung einer Atherosklerose oder einem erhöhten Risiko für Herzinfarkt, rheumatische Erkrankungen oder Diabetes mellitus.

Ein begünstigender Faktor für entzündliche Erkrankungen der Mundhöhle ist vermehrter Zahnbelag (Plaque). Dieser entsteht beispielsweise durch Ansammlung von Nahrungsresten an Stellen, die mechanischem Abrieb beim Kauen oder bei der Zahnreinigung weniger stark ausgesetzt sind. Infolgedessen kommt es dort zu vermehrtem Wachstum von Bakterien, was die Ausbildung von bakteriellen Biofilmen ermöglicht. Durch Einlagerung von Mineralien kann der Zahnbelag zu Zahnstein verkalken.

Durch die Ansiedlung von Bakterien im Bereich des Zahnbelags werden verschiedenste bakterielle Stoffwechsel- und Abbauprodukte freigesetzt. Die Anwesenheit solcher Stoffe ist ein Warnhinweis für eine mögliche Infektion. Das Immunsystem des besiedelten Organismus ist in der Lage, diese Hinweise wahrzunehmen und eine entsprechende Abwehrreaktion einzuleiten. Zu diesem Zweck können Zellen des besiedelten Organismus die Anwesenheit dieser bakteriellen Produkte mittels spezifischer Rezeptoren auf der Zelloberfläche registrieren. Über verschiedenste zelluläre Signalwege und entsprechende Zytokine wird der Warnhinweis dann im Organismus weitergeleitet und es kommt zur Rekrutierung spezialisierter Immunzellen zur Bekämpfung der Infektionserreger. Beispielsweise können einige Immunzellen bestimmte chemische Stoffe und Enzyme freisetzen, die in der Lage sind, Bakterien zu zerstören.

Als entzündliche Erkrankungen der Mundhöhle sind unter anderem Parodontalerkrankungen bekannt. Hierunter fallen beispielweise Entzündungen des Zahnhalteapparats (Parodontium), welche als Parodontitis bezeichnet werden, oder Entzündungen des Zahnfleisches (Gingivitis). Generell wird bei Parodontitis unterschieden zwischen der marginalen Parodontitis, welche vom Zahnfleischsaum ausgeht, und der apikalen Parodontitis, welche von der Zahnwurzelspitze ausgeht. Die Prophylaxe und Behandlung all dieser Erkrankungen stellt ein wichtiges Gebiet der Zahnmedizin dar.

Nach aktueller Klassifikation der Parodontalerkrankungen fallen unter diesen Begriff gingivale Erkrankungen, chronische Parodontitis, aggressive Parodontitis, Parodontitis als Manifestation systemischer Erkrankungen, nekrotisierende Parodontalerkrankungen, Abszesse des Parodonts, Parodontitis im Zusammenhang mit endodontischen Läsionen und entwicklungsbedingte oder erworbene Deformationen und Zustände.

Im Verlauf von Parodontalerkrankungen greift meist eine auf Bakterien zurückzuführende Entzündungsreaktion auf mehrere oder sogar alle Anteile des Parodontiums über. Dabei kommt es zu Gewebedestruktion und -abbau des Parodontiums.

Bei der marginalen Parodontitis zum Beispiel handelt es sich um eine Infektion mit spezifischen strikt anaeroben bzw. mikroaerophilen Erregern, die typischerweise chronisch rezidivierend verläuft. Die wichtigsten Parodontitis-auslösenden Erreger sind: *Porphyromonas gingivalis, Tannerella forsythia, Aggregatibacter actinomycetemcomitans,* alles gramnegative Erreger. Diese Bakterien werden auch als Parodontitis-Markerbakterien bezeichnet. Eine Folge von derartigen chronischen Infektionen ist der langsame Verlust des bindegewebigen Halteapparats des Zahnes (sog. Attachmentverlust), bei langfristigem Voranschreiten der Erkrankung auch der Verlust des knöchernen Zahnhalteapparates des Kiefer-Alveolarknochens.

Das Parodontium ist, wohl aufgrund des andauernden Kontakts mit Mikroorganismen, ein immunologisch sehr reaktionsfreudiges Gewebe, das auf Freisetzung von bakteriellen Produkten der Parodontitis-Erreger stark anspricht. Hierdurch ist zu erklären, dass bei Parodontalerkrankungen anders als bei Infektionen in anderen Kompartimenten des Körpers die Gewebedestruktion hauptsächlich durch die körpereigene Immunreaktion hervorgerufen wird. Dazu gehören der Bindegewebsabbau durch von Immunzellen freigesetzte Enzyme wie z.B. Metalloproteinasen und der Knochenabbau durch Aktivierung von Osteoklasten, die durch Prostaglandine und TNF-alpha sowie einige Interleukine befördert wird.

Zudem produzieren die Parodontitis-Markerbakterien auch Virulenzfaktoren, die ihre Gewebeinvasivität begünstigen und somit zu einer lokalen Verstärkung der Entzündungsreaktion führen. Körpereigene Mechanismen, wie beispielsweise Citrullinierung von Proteinen, fördern die Immunreaktion weiter, was wiederum den Zellverlust auf Seiten des betroffenen Individuums verstärkt.

Als Prophylaxe für Parodontalerkrankungen wird neben häuslicher Mundhygiene die Durchführung von professioneller Zahnreinigung empfohlen, welche dazu dient, vorhandenen Zahnbelag und/oder Zahnstein möglichst vollständig zu entfernen, um eine entzündungsbegünstigende Aktivierung des Immunsystems in physiologischen Grenzen zu halten bzw. zu vermeiden.

In der Zahnmedizin werden Parodontalerkrankungen gemäß mehreren Ansätzen behandelt. Von großer Bedeutung ist die Reinigung der Zahnwurzel und des Zahnschmelzes von Plaque durch mechanische Methoden, auch mittels Ultraschalls und Laseranwendung. Weiterhin können Antiseptika wie z.B. Chlorhexidin mittels stumpfer Kanüle oder auch unter Verwendung von Papierträgern (Periochip^{®}) in den Sulcus gingivalis eingebracht werden. Weiterhin können Antibiotika wie z.B. Minocyclin in den Sulcus gingivalis eingebracht werden. Das Einbringen erfolgt oft mittels eines Trägermaterials, das langsam abgebaut wird und über diesen Zeitraum gleichmäßig den Wirkstoff freisetzt.

Für einen langfristigen Behandlungserfolg müssen die beschriebenen Methoden wiederholt durchgeführt werden. Da diese Verfahren, insbesondere die auf mechanischer Reinigung basierenden, jedoch teilweise schmerzhaft sind, ist die Bereitschaft von Patienten, diese regelmäßigen Wiederholungen zu ermöglichen, oft nicht besonders hoch, was den Behandlungserfolg mindert.

Die in bestimmten Fällen parallel zur Entfernung von Zahnbelag und/oder Zahnstein durchgeführte antiseptische oder antibiotische Behandlung wirkt nicht gezielt gegen die Erreger, welche ursächlich für die Parodontalerkrankung sind, sondern schädigt meist die gesamte Mundflora. Infolgedessen kann zum einen das Gleichgewicht der Mundflora geschädigt werden, so dass es zu einer starken Vermehrung bestimmter Mikroorganismen kommt, die gegen ein Antibiotikum resistent sind. Dies kann weitere Infektionen begünstigen. Zum anderen können beim Einsatz von Antibiotika, und insbesondere bei langfristiger Anwendung, Resistenzen auf Seite der Mikroorganismen entstehen, was derzeit eine der größten Bedrohungen für die öffentliche Gesundheit darstellt. Darüber hinaus werden durch das Abtöten von Bakterien mittels Antibiotika bakterielle Produkte, besonders Abbauprodukte (Bakterientoxine), freigesetzt, die die körpereigene Immunreaktion und damit einhergehend unter Umständen die Gewebedestruktion weiter verstärken und das physiologische Milieu nachhaltig negativ beeinflussen.

Bestimmte Kombinationen von Ionenaustauschermaterialien mit festgelegtem Calcium- und Fluoridionenkonzentrationen wurden für die Behandlung von Parodontitis in SU825074 vorgeschlagen. Eine Wirkung dieser Substanzen bei der Adsorption bakterieller Toxine ist nicht bekannt geworden. WO 2012/123363 A1 beschreibt Materialien zur Behandlung lokaler bakterieller Infektion.

Aufgabe der vorliegenden Erfindung war es nunmehr, verbesserte und insbesondere auch den Patienten schonende Möglichkeiten zur Vorbeugung oder/ und Behandlung von entzündlichen Zuständen oder/und Erkrankungen der Mundhöhle bereitzustellen.

Gelöst wird diese Aufgabe durch die Bereitstellung einer Zusammensetzung, welche im Rahmen der Vorbeugung oder/und Behandlung von entzündlichen Zuständen oder/und Erkrankungen der Mundhöhle bakterielle Produkte, bevorzugt bakterielle Toxine, adsorbiert. Erfindungsgemäß enthält die Zusammensetzung die Substanz Colestyramin (Poly(trimethylammoniomethylsty-rolchlorid-co-divinylbenzol), welche Anionenaustauschergruppierungen aufweist. Diese sind dazu geeignet, bakterielle Produkte, insbesondere toxische bakterielle Produkte wie z.B. Lipopolysaccharide (LPS) oder/und Lipoteichonsäuren (LTA), zu adsorbieren, und ist an der Mundschleimhaut haftfähig, indem sie eine dickflüssige, pastenartige oder gelartige Konsistenz aufweist oder eine derartige Konsistenz bei Benetzung entsteht.

Infolge dieser Adsorption wird zum einen eine Diffusion der toxischen bakteriellen Produkte vom Entzündungsherd in angrenzende Bereiche verhindert. Zum anderen wird durch die Adsorption die Menge der in Lösung vorliegenden toxischen bakteriellen Produkte verringert. Damit wird eine Voraussetzung zur Herstellung des physiologischen Milieus im Entzündungsbereich geschaffen und eine normale Immunreaktion gefördert und gewährleistet.

Somit stellt die vorliegende Erfindung Möglichkeiten bereit, entzündungsfördernde bakterielle Produkte, insbesondere LPS und LTA, durch Bindung zu neutralisieren. In Abwesenheit einer solchen Adsorption bzw. Neutralisierung kommt es zu einer Erhöhung der Konzentration der in Lösung vorliegenden toxischen bakteriellen Produkte, was durch weitere Aktivierung des Immunsystems zu einer Verstärkung der Gewebedestruktion führt. Ein solcher Behandlungsansatz wurde in der Parodontologie bisher nicht verfolgt.

Weiterhin wurde festgestellt, dass es über die Adsorption bzw. Neutralisierung hinaus auch möglich ist, die toxischen bakteriellen Produkte vom Ort der Entzündung zu entfernen, indem das Colestyramin bzw. die Colestyramin-haltige Zusammensetzung nach der Anwendung am Entzündungsort wieder entfernt wird.

Die Adsorption bzw. Neutralisierung oder/und Entfernung der toxischen bakteriellen Produkte vom Ort der Entzündung verbessert generell die Bedingungen für natürliche Regenerationsprozesse. Weiterhin bewirkt ein solches Vorgehen eine konsequente Herabregulation der lokalen Immunreaktion und führt damit zu einer signifikanten Verringerung der Gewebezerstörung, insbesondere durch körpereigene Mechanismen. Dies ist von großer Bedeutung insbesondere für die Behandlung von Parodontalerkrankungen, da der bindegewebige Zahnhalteapparat sich nach Schädigung nur schwer regeneriert.

Generell kann die erfindungsgemäß verwendete Zusammensetzung alleine oder in Kombination mit bekannten Vorbeugungs- oder Behandlungsverfahren eingesetzt werden. Der erfindungsgemäße Einsatz des Adsorptionsmittels Colestyramin ist schmerzfrei für den Patienten, beispielsweise im Gegensatz zur mechanischen Reinigung, und es sind keinerlei Nebenwirkungen zu erwarten. Die Substanz ist seit mehr als 40 Jahren in der Medizin zur Senkung des Cholesterins im Blut und zur Bindung von Gallensalzen im Darm bekannt und verbreitet eingesetzt. Durch eine regelmäßige Anwendung wird der allgemeine Behandlungserfolg positiv beeinflusst und infolgedessen kann die Behandlungsdauer reduziert werden im Vergleich zur Dauer einer Behandlung, welche ausschließlich auf bekannten Behandlungsverfahren basiert. Es ist davon auszugehen, dass diese Faktoren dazu beitragen, dass Patienten sich zuverlässig an ein vom behandelnden Arzt vorgegebenes Therapieschema halten. Somit wird durch die Bereitstellung der Zusammensetzung zur Behandlung von Erkrankungen, insbesondere Parodontalerkrankungen, auch die Patienten-Compliance verbessert.

Weiterhin kann bei erfindungsgemäßer Verwendung des Adsorptionsmittels vermieden werden, dass eine massive, lokale Freisetzung von toxischen bakteriellen Produkten zum Übertritt dieser Produkte in den Blutstrom und damit in den gesamten Körper des Patienten führt. Dieser Vorgang kann die bekannte Folgekaskade auslösen, die über Aktivierung von Mediatoren teilweise autoimmun-destruktiv voranschreitet und zu septischen Erscheinungsformen bis hin zum septischen Schock und Tod des Patienten führen kann.

Es ist heute allgemein anerkannt, dass ein Zusammenhang, zumindest ein Teilzusammenhang zwischen Parodontose und Atherosklerose, speziell der koronaren Herzerkrankung (KHK) und der Herzinfarktinzidenz auch auf der Generalisierung und Chronifizierung von Entzündungen im Mund- und Zahnbereich basiert. Parodontose-Patienten haben ein um 25% erhöhtes KHK-Risiko im Vergleich zu Zahngesunden. Auch ist die Sterblichkeit von KHK-Patienten zweifach höher beim Vorliegen einer Parodontose im Vergleich zu Zahngesunden.

Als Bindeglied zwischen einer chronischen Entzündung im Zahnbereich und der KHK können proinflammatorische und prothrombotische Entzündungsmediatoren fungieren, die in Folge einer lokal erhöhten bakteriellen Toxinlast vermehrt in die Blutbahn eingeschwemmt werden. Dort können sie wesentliche vaskuläre-atherogene Prozesse ankurbeln und unterhalten, die dann letztlich zum Verschluss einer Arterie führen werden. Es existieren preliminäre Ergebnisse, die darauf hinweisen, dass die Sanierung chronischer Inflammationsherde (z.B. Parodontose) auch zur Normalisierung erhöhter Entzündungsmediatoren im Blut und Gewebe führt, die Effizienz einer Statintherapie zur Senkung von LDL steigert und eine Reduktion notwendiger Insulinmengen in der Behandlung eines Diabetes mellitus erlaubt.

Es ist daher zu erwarten, dass die erfindungsgemäße Anwendung von LPS- und LTA-Adsorptionsmaterial im Oralbereich auch unter anderem zur Absenkung der Herzinfarktrate in der Bevölkerung führt.

Ferner wurde festgestellt, dass die erfindungsgemäße Verwendung des Adsorptionsmittels mit den physiologischen Abwehr- und Reparaturmechanismen des Organismus nicht interferiert. Durch die erfindungsgemäße Verwendung werden die schädlichen bakteriellen Produkte zwar in äußerst effektiver Weise entfernt, dagegen wird das physiologische Milieu am Ort der Entzündung, insbesondere im Hinblicke auf die Konzentrationsverhältnisse der körpereigenen Mediatoren und Zytokine, so weit normalisiert, dass die physiologischen Heilungsprozesse ungestört ablaufen können.

Generell resultiert ein entzündlicher Zustand der Mundhöhle, welcher gemäß vorliegender Erfindung behandelt wird, aus einer Infektion oder einer Verletzung der Mundschleimhaut. Als Infektionserreger kommen hier nicht nur Vertreter der natürlichen Mundflora infrage, sondern auch beliebige aus der Umwelt über die Mundöffnung aufgenommene Mikroorganismen.

In einer anderen Ausführungsform der Erfindung resultiert der entzündliche Zustand der Mundhöhle aus einer Infektion mit Bakterien. In einer bevorzugten Ausführungsform handelt es sich um eine Infektion mit gramnegativen Bakterien und/oder grampositiven Bakterien. In einer besonders bevorzugten Ausführungsform handelt es sich um eine Infektion mit einem oder mehreren Bakterien, ausgewählt aus der Gruppe, bestehend aus *Porphyromonas gingivalis, Tannerella forsythia, Aggregatibacter actinomycetemcomitans.*

Die Entstehung einer chronischen Infektion vor Ort kann bei einer allgemeinen Immunabwehrschwäche auch begünstigt werden durch Verletzungen der Mundschleimhaut, sei es beim Kauvorgang oder durch beliebige Verletzungen, auch im Rahmen von Operationen.

In einer Ausführungsform ist die Erkrankung der Mundhöhle eine Parodontalerkrankung. In einer bevorzugten Ausführungsform ist die Parodontalerkrankung eine marginale Parodontitis. In einer besonders bevorzugten Ausführungsform ist die Parodontalerkrankung ausgewählt aus der Gruppe, bestehend aus gingivalen Erkrankungen, chronischer Parodontitis, aggressiver Parodontitis, Parodontitis als Manifestation systemischer Erkrankungen, nekrotisierenden Parodontalerkrankungen, Abszessen des Parodonts, Parodontitis im Zusammenhang mit endodontischen Läsionen und/oder entwicklungsbedingten oder erworbenen Deformationen und Zuständen. Das erfindungsgemäß verwendete Mittel zur Adsorption von bakteriellen Produkten, insbesondere von toxischen bakteriellen Produkten wie z.B. LPS und LTA, weist Anionenaustauschergruppierungen auf. Diese sind in der Lage, LPS und LTA selektiv und effektiv zu binden und somit am Ort der Entzündung aufzunehmen, zu neutralisieren und gegebenenfalls sogar deren Entfernung zu ermöglichen.

Es ist von Vorteil, wenn eine gute Benetzung des LPS oder/und LTA adsorbierenden Colestyramins am Verwendungsort gewährleistet ist und es bei der Anwendung eine möglichst große Oberfläche aufweist. Colestyramin, welches bereits zur Behandlung der Hypercholesterinämie, Bindung von Gallensäuren im Darm und auch von chologener Diarrhö eingesetzt wird, liegt für die bekannten Verwendungen als Substanz in Form eines feinen hygroskopischen Pulvers vor. Es ist ein enzymatisch nicht zersetzbares, wasserunlösliches Makromolekül, welches in der Schulmedizin seit über 40 Jahren bekannt ist, im Darm nicht resorbierbar ist und damit nicht verstoffwechselt wird. Bisher ist es in Form von Granulaten, Pulver oder Kautabletten für perorale Einnahme im Handel.

Erfindungsgemäß ist es möglich, das in der Zusammensetzung vorhandene Colestyramin in Kombination mit einem oder mehreren weiteren Wirkstoffen zu verwenden. Bezüglich der Auswahl solcher weiterer Wirkstoffe bestehen keinerlei Limitierungen. Ein Fachmann kann beliebige weitere Wirkstoffe mit dem erfindungsgemäß verwendeten Adsorptionsmittel kombinieren, so lange die vorgesehene Wirksamkeit des Adsorptionsmittels hierdurch nicht dergestalt beeinträchtigt wird, dass die erfindungsgemäß vorgesehene Adsorption toxischer bakterieller Produkte verhindert wird.

In einer bevorzugten Ausführungsform ist der eine oder sind die mehreren weiteren Wirkstoffe ausgewählt aus der Gruppe, bestehend aus antibakteriellen Wirkstoffen, entzündungshemmenden Wirkstoffen, schmerzhemmenden Wirkstoffen, regenerierenden Wirkstoffen oder Mischungen davon. Besonders bevorzugt sind hierbei Antibiotika, Silberionen, Zinkoxid, Povidon-Iod, Aktivkohle, Ibuprofen, Acetylsalicylsäure, Diclofenac, Lokalanästhetika wie z.B. Lidocain oder Benzocain, Dexpanthenol oder Mischungen davon.

Konventionelle Therapie von entzündlichen Zuständen oder/und Erkrankungen der Mundhöhle umfasst oft zumindest unterstützend eine antibiotische oder antiseptische Behandlung. Eine Antibiotikabehandlung wirkt meist nicht selektiv gegen die pathogenen Mikroorganismen, die die zu behandelnde Erkrankung verursachen, sondern auch gegen die natürliche Mundflora. Eine Schädigung des normalerweise in der Mundhöhle vorliegenden natürlichen Gleichgewichts der Mundflora kann wiederum negative Auswirkungen auf den Therapieerfolg haben, beispielsweise indem Infektionen durch opportunistische Erreger begünstigt werden. Weiterhin besteht bei Antibiotikabehandlungen generell das Problem einer Resistenzentwicklung, welche den Therapieerfolg gefährden kann. Dennoch ist es bei der herkömmlichen Behandlung von entzündlichen Zuständen oder/und Erkrankungen der Mundhöhle oft nötig, Antibiotika oder andere antibakterielle Wirkstoffe einzusetzen, um das körpereigene Immunsystem bei der Bekämpfung einer krankheitsverursachenden bakteriellen Besiedelung zu unterstützen.

Im Rahmen der vorliegenden Erfindung wird es durch erfindungsgemäße Verwendung von Colestyramin durch Erniedrigung der Toxinlast oftmals ermöglicht, auf die Verwendung von Antibiotika oder anderen antibakteriellen Wirkstoffen zu verzichten, beziehungsweise deren Verwendung weitgehend zu reduzieren.

Durch erfindungsgemäße Verwendung einer Zusammensetzung enthaltend Colestyramin wird das körpereigene Immunsystem unterstützt. Unter bestimmten Voraussetzungen ist es beispielsweise auch denkbar, dass durch Neutralisierung immunaktivierender bakterieller Produkte das Absterben von Immunzellen verringert werden kann und auf diese Weise mehr Immunzellen zur Bekämpfung der bakteriellen Erreger zu Verfügung stehen. Durch eine solche Unterstützung wird das Immunsystem in die Lage versetzt, die bakterielle Infektion (erhöhte Toxinlast) besser zu kontrollieren und die Verabreichung von Antibiotika und/oder anderen antibakteriell wirkenden Mitteln bei der Behandlung eines entzündlichen Zustands oder/und einer Erkrankung der Mundhöhle zumindest zu reduzieren.

Es kann auch möglich sein, vollständig auf den unterstützenden Einsatz von Antibiotika zu verzichten, wenn der behandelnde Arzt erkennt, dass sich durch mechanische Reinigung und/oder Verwendung der erfindungsgemäßen Zusammensetzung ein ausreichender Behandlungserfolg einstellt.

Durch Verzicht auf den Einsatz von Antibiotika und/oder Reduzierung deren Verwendung ist es möglich, die nachteiligen Effekte dieser Behandlungsoptionen zu überwinden bzw. zu verringern. Insbesondere wird durch das erfindungsgemäß verwendete Colestyramin weder das Gleichgewicht der Mundflora negativ beeinflusst, noch wird der Antibiotikaresistenzentwicklung von Bakterien Vorschub geleistet. In diesem Zusammenhang ist insbesondere zu beachten, dass eine Resistenzentwicklung gegen die erfindungsgemäße Neutralisierung toxischer bakterieller Produkte nicht erwartet werden kann.

In einer anderen Ausführungsform der vorliegenden Erfindung und soweit dies notwendig erscheint, kann in der Zusammensetzung Colestyramin in Kombination mit einem Antibiotikum und/oder antibakteriellen Mitteln eingesetzt werden. Im Rahmen der vorliegenden Erfindung konnte festgestellt werden, dass die Behandlung entzündlicher Erkrankungen der Mundhöhle auch durch ein solches Vorgehen positiv beeinflusst werden kann.

Die gezielte Abtötung von Bakterien trägt zwar zur Bekämpfung der entzündungsauslösenden Ursache bei, führt jedoch auch zu einer massiven Freisetzung toxischer bakterieller Produkte, insbesondere bei Einsatz bakterizider Mittel. Diese bakteriellen Produkte verstärken wiederum die der Gewebedestruktion zugrundeliegenden körpereigenen Mechanismen. Ganz im Gegenteil zur bisherigen Situation kann durch die erfindungsgemäß eingesetzte Zusammensetzung eine Neutralisierung dieser bakteriellen Produkte erreicht werden, bevor eine (Über-)Aktivierung des Immunsystems erfolgen kann. Somit kann vermieden werden, dass eine verstärkte Gewebedestruktion auftritt und/oder der Heilungsprozess durch die Freisetzung von toxischen bakteriellen Produkten in der Folge einer Antibiose auf sonstige Weise negativ beeinflusst wird. Weiterhin kann vermieden werden, dass die toxischen bakteriellen Produkte in die Blutbahn übertreten und nachteilige systemische Effekte bis hin zum septischen Schock verursachen oder im chronischen Verlauf zur beschleunigten Atherosklerose, zu Herzinfarkt, einem Diabetes mellitus oder anderen Krankheiten mit entzündlicher Komponente führen.

Die Kombination von Colestyramin mit antibakteriellen Mitteln ist insbesondere bei der Verwendung von bakteriziden Mitteln und/oder bei Vorliegen von hoher Bakteriendichte, wie es bei Parodontalkrankheiten der Fall ist, von Vorteil.

Die erfindungsgemäß verwendete Zusammensetzung kann auch mit Produkten, die zur Pflege, Reinigung und/oder Behandlung der Mundhöhle verwendet werden, kombiniert werden. Beispielsweise kann das Colestyramin als Zusatzstoff in Zahnreinigungsprodukten wie z.B. Zahnpasta oder Zahngel verwendet werden.

Bedingt durch die Funktion der Speicheldrüsen weist die Mundhöhle ein ständig feuchtes Milieu auf, wobei die Oberfläche der Mundhöhle, insbesondere Mundschleimhaut und Zahnoberflächen, für gewöhnlich ständig von einem dünnen Flüssigkeitsfilm bedeckt sind. Bei einer lokalen Applikation von therapeutischen Mitteln in der Mundhöhle ergibt sich somit generell das Problem, dass diese nach der Applikation bedingt durch diesen Flüssigkeitsfilm und mechanische Reibung, welche durch Mundbewegungen erzeugt wird, wieder vom Aufbringungsort entfernt werden. Die hieraus resultierende Problematik einer unzureichenden Verweildauer des aufgebrachten Mittels an der jeweiligen zu behandelnden Stelle kann auch bei der erfindungsgemäßen Verwendung von Colestyramin bestehen.

Dieses Problem wird erfindungsgemäß überwunden, indem die erfindungsgemäß eingesetzte Zusammensetzung in einer Form verwendet wird, welche an Schleimhaut haftfähig ist. Dies wird erfindungsgemäß erreicht, indem eine Zusammensetzung mit dickflüssiger, pastenartiger oder gelartiger Konsistenz verwendet wird. Es ist auch möglich, dass eine solche Konsistenz erst bei Benetzung der Zusammensetzung entsteht. Eine solche Benetzung kann erfolgen durch in der Mundhöhle vorhandene Flüssigkeit, aber auch durch von außen zu applizierende Flüssigkeit.

In einer besonders bevorzugten Ausführungsform umfasst die Zusammensetzung der Erfindung ein Geliermittel oder eine Gelbasis. Derartige Geliermittel können ausgewählt sein aus der Gruppe, bestehend aus Alginaten, Polyacrylsäure, Carboxymethylcellulose, Tragantgummi, Siliciumdioxid, Gelatine, Methylcellulose, Poloxameren oder Povidon, sind aber nicht hierauf beschränkt. Dem Fachmann sind weitere zu diesem Zweck geeignete Hilfsmittel bekannt, beispielsweise aus Standardwerken wie "Remington's Pharmaceutical Sciences".

Die Verwendung der oben beschriebenen Zusammensetzungen speziell in der Mundhöhle ist besonders vorteilhaft, da die beschriebene Haftfähigkeit durch die räumliche Enge bestimmter Applikationsbereiche, wie z.B. in Zahnzwischenräumen oder Zahnfleischtaschen, noch verstärkt wird. Auf diese Weise kann dort eine weiter erhöhte Verweildauer des Colestyramins erreicht werden. Insbesondere bei Parodontalerkrankungen sind diese Applikationsbereiche von besonderem Interesse. Auch der die Erkrankung auslösende Zahnbelag bildet sich bevorzugt an diesen Stellen.

Bevorzugt wird die erfindungsgemäße Zusammensetzung in einer Darreichungsform bereitgestellt, die ausgewählt ist aus der Gruppe, bestehend aus einer Paste wie z.B. einer Zahnpasta, einem Gel wie z.B. einem Zahngel oder einer sprühbaren Flüssigkeit wie z.B. einem Spray. Möglich ist auch eine Bereitstellung unter Verwendung von anderen Materialen wie Hohlfasern, Membranen, Flachmembranen, Schwämmen, Filtermaterialien, Partikeln, porösen Partikeln, Beads, Granulaten oder Pulvern, auf welche Colestyramin aufgebracht oder mit welchen es gemischt wird, solange eine solche Darreichungsform die erfindungsgemäße dickflüssige, pasten- oder gelartige Konsistenz aufweist, oder diese bei Benetzung entsteht. Diese Darreichungsformen können in verschiedensten Applikatoren bereitgestellt werden, je nach Konsistenz beispielsweise in Spendern oder Sprühdosierern, wie z.B. einer Sprühdose oder einem Pulversprüher. Ebenfalls möglich ist eine Bereitstellung in Einlagepads, welche geeignet sind zur Anwendung im Schleimhautbereich, wobei derartige Pads z.B. auch in Zahnfleischtaschen eingeführt werden können.

In einer bevorzugten Ausführungsform umfasst die Zusammensetzung gemäß der vorliegenden Erfindung Colestyramin in einer Konzentration von 5 Gew.-% bis 50 Gew.-%, bevorzugt von 5 Gew.-% bis 40 Gew.-% oder von 15 Gew.-% bis 40 Gew.-% oder stärker bevorzugt von etwa 15 Gew.-%, jeweils bezogen auf das Gesamtgewicht der Zusammensetzung.

Zusammensetzungen gemäß der vorliegenden Erfindung können weiterhin beliebige bekannte zusätzliche Trägermaterialen und Hilfsstoffe umfassen.

Die erfindungsgemäße Zusammensetzung kann gemäß vorliegender Erfindung im Schleimhautbereich der Mundhöhle angewendet werden. Insbesondere bevorzugt ist eine Anwendung am Zahnfleisch und/oder dem Zahnfleischsaum bzw. dem Sulcus gingivalis. Besonders bevorzugt ist eine Einbringung in schwer zugängliche Stellen in der Mundhöhle, wie z.B. Zahnfleischtaschen oder in Zahnzwischenräume.

Die Anwendung der erfindungsgemäßen Zusammensetzung kann vor, während und/oder nach anderen Behandlungsverfahren erfolgen.

Neben einer Anwendung zu Zwecken der Therapie kann die erfindungsgemäße Zusammensetzung auch prophylaktisch eingesetzt werden. Die Applikation kann durch den behandelnden Arzt und/oder den Patienten erfolgen.

Bei Verwendung bestimmter Ausgestaltungen der Zusammensetzung ist es nicht einmal nötig, diese wieder über die Mundöffnung zu entfernen, z. B. durch Ausspülen. Eine Entfernung kann auch über das Verdauungssystem erfolgen. Hierfür ist es vorteilhaft, dass Colestyramin ausreichend inert für den Magen-Darm-Trakt ist und nicht resorbiert wird, was seine sehr gute Bioverträglichkeit garantiert.

Die spezifische Ausgestaltung der erfindungsgemäßen Zusammensetzung zur Verwendung in der Mundhöhle liegt im Können des Fachmanns, besonders einfach kann Colestyramin allerdings in eine Zahnpasta integriert werden, welche vorbeugend oder therapeutisch bei der täglichen Mundhygiene eingesetzt werden kann. Eine solche Zahnpasta, welche Colestyramin als aktiven Inhaltsstoff, ggf. in Kombination mit weiteren Wirkstoffen enthält, ist daher ein weiterer Gegenstand der vorliegenden Erfindung.

## Patentansprüche

1. Zusammensetzung zur Verwendung für die Adsorption oder/und Eliminierung bakterieller Toxine, insbesondere bakterieller Lipopolysaccharide (LPS) oder/und Lipoteichonsäuren (LTA), im Rahmen der Vorbeugung oder/und Behandlung von entzündlichen Zuständen oder/und Erkrankungen der Schleimhäute im Bereich der Mundhöhle, wobei der entzündliche Zustand der Mundhöhle aus einer Infektion oder einer Verletzung der Mundschleimhaut resultiert oder die entzündliche Erkrankung der Mundhöhle eine Parodontalerkrankung ist,
**dadurch gekennzeichnet, dass** die Zusammensetzung als bakterielle Toxine absorbierende oder/und eliminierende Substanz Colestyramin enthält und die Zusammensetzung an der Mundschleimhaut haftfähig ist, indem sie eine dickflüssige, pastenartige oder gelartige Konsistenz aufweist oder eine derartige Konsistenz bei Benetzung entsteht.

2. Zusammensetzung zur Verwendung nach Anspruch 1,
**dadurch gekennzeichnet, dass** die Parodontalerkrankung ausgewählt ist aus gingivalen Erkrankungen, chronischer Parodontitis, aggressiver Parodontitis, Parodontitis als Manifestation systemischer Erkrankungen, nekrotisierenden Parodontalerkrankungen, Abszessen des Parodonts, Parodontitis im Zusammenhang mit endodontischen Läsionen und entwicklungsbedingten oder erworbenen Deformationen und Zuständen.

3. Zusammensetzung zur Verwendung nach Anspruch 1 oder 2,
**dadurch gekennzeichnet, dass** die pharmazeutische Zusammensetzung ein Geliermittel oder eine Gelbasis umfasst.

4. Zusammensetzung zur Verwendung nach Anspruch 3,
**dadurch gekennzeichnet, dass** das Geliermittel ausgewählt ist aus der Gruppe, bestehend aus Alginaten, Polyacrylsäure, Carboxymethylcellulose, Tragantgummi, Siliciumdioxid, Gelatine, Methylcellulose, Poloxameren oder Povidon.

5. Zusammensetzung zur Verwendung nach einem der Ansprüche 1 bis 4, **dadurch gekennzeichnet, dass** die pharmazeutische Zusammensetzung in einer Darreichungsform vorliegt, die ausgewählt ist aus der Gruppe, bestehend aus einer Paste wie z.B. einer Zahnpasta, einem Gel wie z.B. einem Zahngel oder einer sprühbaren Flüssigkeit wie z.B. einem Spray.

6. Zusammensetzung zur Verwendung nach einem der Ansprüche 1 bis 5, **dadurch gekennzeichnet, dass** sie einen oder mehrere weitere Wirkstoffe ausgewählt aus der Gruppe, bestehend aus antibakteriellen Wirkstoffen, entzündungshemmenden Wirkstoffen, schmerzhemmenden Wirkstoffen, regenerierenden Wirkstoffen oder Mischungen davon, enthält.

7. Zusammensetzung zur Verwendung oder nach Anspruch 6,
**dadurch gekennzeichnet, dass** der eine oder die mehreren weiteren Wirkstoffe ausgewählt sind aus der Gruppe, bestehend aus einem Antibiotikum, Silberionen, Zinkoxid, Povidon-Iod, Aktivkohle, Ibuprofen, Acetylsalicylsäure, Diclofenac, einem Lokalanästhetikum wie z.B. Lidocain oder Benzocain, Dexpanthenol und Mischungen davon.

8. Zusammensetzung zur Verwendung nach einem der vorhergehenden Ansprüche,
**dadurch gekennzeichnet, dass** sie Colestyramin integriert in eine Zahnpastabasis aufweist.

9. Zahnpasta,
**dadurch gekennzeichnet, dass** sie Colestyramin als aktiven Inhaltsstoff und ggf. mit weiteren Wirkstoffen kombiniert aufweist.

## Claims

1. Composition for use for the adsorption and/or elimination of bacterial toxins, in particular bacterial lipopolysaccharides (LPS) and/or lipoteichoic acids (LTA), in the context of the prevention and/or treatment of inflammatory states or/and diseases of the mucous membranes in the region of the oral cavity, the inflammatory state of the oral cavity resulting from an infection or injury to the oral mucosa, or the inflammatory disease of the oral cavity being a periodontal disease,
**characterized in that** the composition contains colestyramine as a bacterial-toxin absorbing and/or eliminating substance, and the composition is capable of adhering to the oral mucosa by having a viscous, paste-like or gel-like consistency or by such a consistency forming during wetting.

2. Composition for use according to claim 1,
**characterized in that** the periodontal disease is selected from gingival diseases, chronic periodontitis, aggressive periodontitis, periodontitis as a manifestation of systemic diseases, necrotizing periodontal diseases, abscesses of the periodontium, periodontitis in connection with endodontic lesions and developmental conditions or acquired deformations and states.

3. Composition for use according to claim 1 or 2,
**characterized in that** the pharmaceutical composition comprises a gelling agent or a gel base.

4. Composition for use according to claim 3,
**characterized in that** the gelling agent is selected from the group consisting of alginates, polyacrylic acid, carboxymethyl cellulose, gum tragacanth, silicon dioxide, gelatin, methylcellulose, poloxamers or povidone.

5. Composition for use according to any of claims 1 to 4,
**characterized in that** the pharmaceutical composition is present in a dosage form selected from the group consisting of a paste such as a toothpaste, a gel such as a tooth gel, or a sprayable liquid such as a spray.

6. Composition for use according to any of claims 1 to 5,
**characterized in that** it contains one or more further active substances selected from the group consisting of antibacterial active substances, antiinflammatory active substances, pain-inhibiting active substances, regenerating active substances, or mixtures thereof.

7. Composition for use or according to claim 6,
**characterized in that** the one or more further active substances are selected from the group consisting of an antibiotic, silver ions, zinc oxide, povidone iodine, activated carbon, ibuprofen, acetylsalicylic acid, diclofenac, a local anesthetic such as lidocaine or benzocaine, dexpanthenol, and mixtures thereof.

8. Composition for use according to any of the preceding claims,
**characterized in that** it has colestyramine integrated into a toothpaste base.

9. Toothpaste,
**characterized in that** it has colestyramine as an active ingredient and optionally combined with further active substances.

## Revendications

1. Composition à utiliser pour l'adsorption ou/et l'élimination de toxines bactériennes, en particulier de lipopolysaccharides (LPS) bactériens ou/et d'acides lipoteichoniques (LTA), dans le cadre de la prévention ou/et du traitement d'états inflammatoires ou/et de maladies des muqueuses dans la région de la cavité buccale, dans lequel l'état inflammatoire de la cavité buccale résulte d'une infection ou d'une lésion de la muqueuse buccale ou la maladie inflammatoire de la cavité buccale est une maladie parodontale,
**caractérisée en ce que** la composition contient de la colestyramine en tant que substance absorbant ou/et éliminant les toxines bactériennes et **en ce que** la composition peut adhérer à la muqueuse buccale en présentant une consistance épaisse, pâteuse ou gélatineuse ou en produisant une telle consistance par mouillage.

2. Composition à utiliser selon la revendication 1,
**caractérisée en ce que** la maladie parodontale est choisie parmi les maladies gingivales, la parodontite chronique, la parodontite agressive, la parodontite en tant que manifestation de maladies systémiques, les maladies parodontales nécrosantes, les abcès du parodonte, la parodontite associée à des lésions endodontiques et les déformations et conditions développementales ou acquises.

3. Composition à utiliser selon la revendication 1 ou 2,
**caractérisée en ce que** la composition pharmaceutique comprend un agent gélifiant ou une base de gel.

4. Composition à utiliser selon la revendication 3,
**caractérisée en ce que** l'agent gélifiant est choisi dans le groupe constitué par les alginates, l'acide polyacrylique, la carboxyméthylcellulose, la gomme adragante, la silice, la gélatine, la méthylcellulose, les poloxamères ou la povidone.

5. Composition à utiliser selon l'une des revendications 1 à 4,
**caractérisée en ce que** la composition pharmaceutique est sous une forme d'administration choisie dans le groupe constitué par une pâte telle qu'un dentifrice, un gel tel qu'un gel dentaire ou un liquide pulvérisable tel qu'un spray.

6. Composition à utiliser selon l'une des revendications 1 à 5,
**caractérisée en ce qu'**elle contient un ou plusieurs autres principes actifs choisis dans le groupe constitué par les principes actifs antibactériens, les principes actifs anti-inflammatoires, les principes actifs analgésiques, les principes actifs régénérateurs ou leurs mélanges.

7. Composition à utiliser ou selon la revendication 6,
**caractérisée en ce que** ledit un ou lesdits plusieurs autres ingrédients actifs sont choisis dans le groupe constitué d'un antibiotique, d'ions argent, d'oxyde de zinc, de povidone iodée, de charbon actif, d'ibuprofène, d'acide acétylsalicylique, de diclofénac, d'un anesthésique local tel que la lidocaïne ou la benzocaïne, de dexpanthénol et de leurs mélanges.

8. Composition à utiliser selon l'une des revendications précédentes,
**caractérisée en ce qu'**elle comprend de la colestyramine intégrée dans une base de dentifrice.

9. Pâte dentifrice,
**caractérisé en ce qu'**il comprend de la colestyramine en tant qu'ingrédient actif et, le cas échéant, en combinaison avec d'autres substances actives.
